# EUROPEAN PATENT SPECIFICATION

(11) **EP 1 363 886 B1**
(45) Date of publication and mention of the grant of the patent: **04.06.2008**
(21) Application number: 02719829.0
(22) Date of filing: 14.02.2002
(51) Int. Cl.: C07D 213/50, B01J 21/06, B01J 23/02, B01J 23/04

(54) **PROCESS AND CATALYST FOR THE PREPARATION OF ACETYLPYRIDINES**
VERFAHREN UND KATALYSATOR ZUR HERSTELLUNG VON ACETYLPYRIDINEN
PROCEDE ET CATALYSEUR DE PREPARATION D'ACETYLPYRIDINES

(30) Priority: 19.02.2001 EP 01103953; 26.11.2001 US 332546 P
(43) Date of publication of application: 26.11.2003
(73) Proprietor: Lonza AG, 4052 Basel (CH)
(72) Inventor: CHUCK, Roderick, John, CH-3902 Brig-Glis (CH)
(86) International application number: PCT/EP2002/001533
(87) International publication number: WO 2002/066433

(56) References cited:
- EP-A- 0 352 674
- WO-A-98/50374
- GB-A- 1 249 079
- GIRARDOT, MARC ET AL: "Direct Conversion of Heteroaromatic Esters to Methyl Ketones with Trimethylaluminum: Nonsymmetrically Disubstituted 1,2,4,5-Tetrazines" JOURNAL OF ORGANIC CHEMISTRY., vol. 63, no. 26, - 1998 pages 10063-10068, XP002203300 AMERICAN CHEMICAL SOCIETY. EASTON., US ISSN: 0022-3263

## Description

The invention relates to a process for the production of acetylpyridines of formula 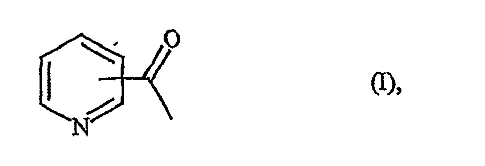 in particular 3-acetylpyridine.

3-Acetylpyridine is assuming increasing importance in the pharmaceutical and fine chemical industry as an intermediate and a building block Up to the present, 3-acetylpyridine has been prepared using often multistage processes starting from nicotinic acid, or from other starting materials which are not readily available (for example 3-ethylpyridine). Instead of using a heteroaromatic acid, it is possible to use derivatives. This is particularly useful when the acid is non-volatile and/or has a tendency to decompose at higher temperatures. Thus ethyl nicotinate was condensed with acetic acid at 520 °C to give 37% yield of 3-acetylpyridine (Houben-Weyl, Vol. VI/2a, pp. 632-633). Recently a process for the production of 3-acetylpyridine has been described in EP-A-0 352 674, which utilises a catalyst based on titanium dioxide and an alkali or alkaline earth metal oxide or hydroxide. Using the methyl ester of nicotinic acid and acetic acid and a catalyst composed of 98% titanium dioxide (as anatase) and 2% sodium oxide, selectivities between 54% and 60% were reported. Pyridine was produced as a side product through decarboxylation of the nicotinate in amounts between 29 and 41%. Various other methods have been described which do not lead to satisfactory results (cf. EP-A-0 352 674).

The purpose of the present invention is to improve the selectivity of the state-of-the-art processes for 3-acetylpyridine, thus avoiding or reducing the losses caused by the formation of large quantities of unwanted side-products.

According to the invention, this has been accomplished by the process of claim 1.

It has been found that in the gas-phase reaction of pyridinecarboxylic esters of formula 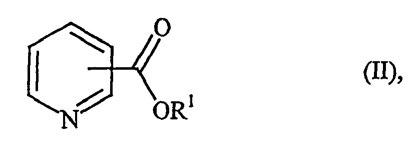 wherein R¹ is C₁₋₆alkyl, with acetic acid to give acetylpyridines (1) the selectivity of the titanium dioxide-based catalysts of EP-A-0 352 674 can be substantially improved by employing a high-porosity alumina-silica support having an apparent porosity of at least 50%, as determined by the Archimedes method.

Preferably, the process according to the invention is carried out with a C₁₋₆-alkyl nicotinate as starting material to give 3-acetylpyridine.

It has further been found that by employing higher boiling pyridinecarboxylic esters (II) the ease of separation of the reaction products can be increased. Lower esters of e. g. nicotinic acid have boiling points similar to those of the desired product, which results in separation difficulties in distillation if the conversion is less than 100%. Use of higher boiling (and more stable) esters largely avoids this problem. Examples of this are the butyl, pentyl or hexyl (including the isomers such as isobutyl, *sec*-butyl, isopentyl etc.) esters of nicotinic acid instead of methyl, ethyl or propyl esters, whose boiling points at atmospheric pressure differ only by 3-4 K (ethyl) and 15-16 K (methyl and propyl), respectively, from that of 3-acetylpyridine. The difference in boiling point between butyl nicotinate and 3-acetylpyridine at atmospheric pressure is 32 K.
Preferably, pyridine carboxylic esters (II) having a boiling point (at atmospheric pressure) exceeding that of the product acetylpyridine (I) by more than 20 K are used as starting materials.

The preferred reaction temperature is 350 to 450 °C.

Advantageously, the reaction is carried out in the presence of water and using an excess of acetic acid.

The weight ratio of alumina to silica in the catalyst support is advantageously between 70:30 and 90:10, preferably between 75:25 and 85:15.

Preferably, the apparent porosity of the catalyst support is between 60 and 70%.

The packing density of the catalyst support is preferably lower than 1000 kg/m³, more preferably between 600 and 800 kg/m³.

Preferably, the titanium dioxide content of the catalyst is 5 to 20 wt. percent, based on the weight of the support.

The catalyst may be prepared by a process comprising the steps of (i) impregnating an alumina-silica support having an apparent porosity of at least 50% with a solution of titanium tetrachloride in aqueous hydrochloric acid to obtain a first catalyst precursor, (ii) drying, (iii) calcining, (iv) impregnating the calcined first catalyst precursor with a solution or suspension of a hydroxide and/or oxide of an alkali metal and/or alkaline earth metal to obtain a second catalyst precursor, (v) drying and (vi) calcining the dried second catalyst precursor to obtain the final catalyst.

The impregnation of the support is not limited to the use of titanium tetrachloride. Thus either other soluble salts of titanium, or even a finely-divided slurry of titanium oxide can be used. It is also possible to replace the hydroxides or oxides of alkali or alkaline earth metals by suitable precursors, e. g. salts of said metals which decompose on heating.

Advantageously, the ratio of alumina to silica in the support is between 70:30 and 90:10, preferably between 75:25 and 85:15.

The apparent porosity of the catalyst support is preferably between 60 and 70%.

Preferably, the titanium dioxide content of the catalyst is 5 to 20 wt. percent, based on the weight of the support.

The following non-limiting examples will illustrate the process of the invention and the preparation of the catalyst.

### Example 1

### Preparation of Catalyst:

For the preparation of the catalyst porous silica-alumina spheres are utilised as a support. A suitable material is supplied by the Norton Chemical Process Products Corporation of Akron, Ohio with the following typical specification:

| | |
|---|---|
| Size and Shape: | 4 mm ∅ spheres |
| Surface Area: | 12 m²/g |
| Apparent Porosity: | 65% |
| Packing Density: | 710 kg/m³ |
| Total Pore Volume (by Hg porosimetry): | 0.5 ml/g |
| Al₂O₃: | 79-81% |
| SiO₂: | 17-19% |

An aqueous solution of titanium tetrachloride (22% expressed as TiO₂, 0.21 mol, 16 g) was prepared by adding 23 ml (40 g, 0.21 mol) of TiCl₄ to a mixture of 15 ml of concentrated hydrochloric acid and 60 ml of demineralised water. The final solution was stirred for several minutes and cooled down.
The above silica-alumina spheres (250 g) were impregnated in a rotating glass vessel or metal drum using the wet incipient technique by spraying the TiCl₄ solution at 25-30 °C. The catalyst precursor was subsequently dried under vacuum (bath temperature: 90-95 °C) for 1 h, then at 120 °C for 12 h and finally calcined at 400 °C for 12 h under a stream of air (500 ml/min). An analysis gave 7% Ti.
The calcined spheres were again placed in a rotating drum and sprayed with dilute sodium hydroxide solution (1.5 g NaOH as a 5% aqueous solution).
The catalyst was dried at 120°C for 12 h and then calcined for 1 h at 500 °C.

### Example 2

### Preparation of 3-acetylpyridine

An electrically heated tubular reactor with an inner diameter (i. d.) of 12 mm was filled with 15 ml (12 g) of catalyst from example 1. Over a period of 12 h, a mixture of 17.9 g butyl nicotinate, 32 g water and 125 g acetic acid was metered using a precision pump to the reactor operating at 410 °C. From the reaction mixture, 8.9 g of 3-acetylpyridine, 0.9 g of pyridine and 1.3 g of butyl nicotinate were obtained. This corresponded to a yield of 73% 3-acetylpyridine at a butyl nicotinate conversion of 93% (selectivity 78%). The selectivity of pyridine formation was 11 %.

### Comparative Example 1

**Preparation of 3-acetylpyridine using catalyst described in** EP-A-0 352 674

An electrically heated tubular reactor with an i. d. of 12 mm was filled with 15 ml (≈15 g) of catalyst prepared according to EP-A-0 352 674, example 1. Over a period of 60 h, a mixture of methyl nicotinate (78 g), water (149 g) and acetic acid (523 g) was metered using a precision pump to the reactor operating at 410 °C. From the reaction mixture, 30.5 g of 3-acetylpyridine, 8.5 g of pyridine and 9.0 g of methyl nicotinate were obtained. This corresponded to a yield of 45% 3-acetylpyridine at a methyl nicotinate conversion of 88% (selectivity 50%). The selectivity of pyridine formation was 19%.

### Comparative Example 2

**Preparation of 3-acetylpyridine using catalyst described in** EP-A-0 352 674

An electrically heated tubular reactor with an i. d. of 12 mm was filled with 15 ml (≈15 g) of catalyst prepared according to EP-A-0 352 674, example 1. Over a period of 63 h, a mixture of butyl nicotinate (104 g), water (162 g) and acetic acid (606 g) was metered using a precision pump to the reactor operating at 405 °C. From the reaction mixture, 30 g of acetyl pyridine, 3 g of pyridine and 26 g of butyl nicotinate were obtained. This corresponded to a yield of 43% 3-acetylpyridine at a butyl nicotinate conversion of 75% (selectivity 58%). The selectivity of pyridine formation was 5%.

### Example 3

### Preparation of 3-acetylpyridine

A tubular reactor with an i. d. of 23.5 mm was filled with 270 ml (≈227 g) of catalyst from example 1. A mixture of butyl nicotinate (30 g/h), water (54 g/h) and acetic acid (210 g/h) was fed over an evaporator using a metering pump to the tubular reactor heated by a salt bath. A small flow of nitrogen (11/h) guaranteed the transport of the vapours. The temperature of the bath was 410 °C. The reaction vapours were condensed in an aqueous circulation system. After a total running time of 35 h, the reaction solution was collected. A portion of this solution corresponding to a total of 710 g of butyl nicotinate was worked up to isolate the product. 81% of butyl nicotinate was converted to give 293 g of 3-acetylpyridine, corresponding to a selectivity of 75% and a yield of 61%. The selectivity of pyridine formation was 4%.

## Claims

1. A process for the preparation of an acetylpyridine of the formula 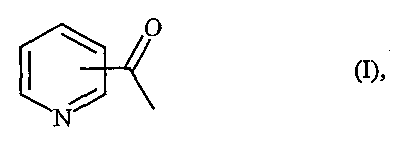 by reacting a pyridinecarboxylic ester of the formula 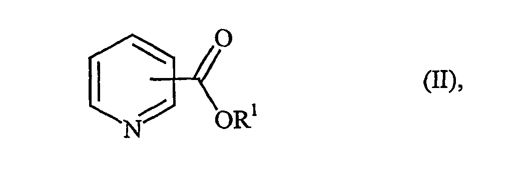 where R¹ is C₁₋₆-alkyl, with acetic acid in the gas phase in the presence of a catalyst whose active material comprises titanium dioxide and at least one alkali or alkaline earth metal oxide, wherein the catalyst comprises an alumina-silica support having an apparent porosity of at least 50%.

2. The process of claim 1, wherein the acetylpyridine (I) prepared is 3-acetylpyridine and the pyridinecarboxylic ester (II) is a C₁₋₆-alkyl nicotinate.

3. The process of claim 1 or 2, wherein the pyridinecarboxylic ester (II) has a boiling point at atmospheric pressure which is more than 20 K higher than the boiling point of the acetylpyridine (I).

4. The process of any one of claims 1 to 3, wherein the reaction temperature is between 350 and 450 °C.

5. The process of any one of claims 1 to 4, wherein the weight ratio of alumina to silica in the catalyst support is between 70:30 and 90:10, preferably between 75:25 and 85:15.

6. The process of any one of claims 1 to 5 wherein the apparent porosity of the catalyst support is between 60 and 70%.

7. The process of any one of claims 1 to 6 wherein the packing density of the catalyst support is between 600 and 1000 kg/m³, preferably between 600 and 800 kg/m³.

8. The process of any one of claims 1 to 7 wherein the titanium dioxide content of the catalyst is 5 to 20 wt. percent based on the weight of the support.

9. The process of any one of claims 1 to 4, wherein the catalyst has been obtained by
(i) impregnating an alumina-silica support having an apparent porosity of at least 50% with a solution of titanium tetrachloride in aqueous hydrochloric acid to obtain a first catalyst precursor,
(ii) drying,
(iii) calcining,
(iv) impregnating the calcined first catalyst precursor with a solution or suspension of a hydroxide and/or oxide of an alkali metal and/or alkaline earth metal to obtain a second catalyst precursor,
(v) drying and
(vi) calcining the dried second catalyst precursor.

10. The process of claim 9 wherein the weight ratio of alumina to silica in the support is between 70:30 and 90:10, preferably between 75:25 and 85:15.

11. The process of claim 9 or 10, wherein the apparent porosity of the catalyst support is between 60 and 70%.

12. The process of any one of claims 9 to 11, wherein the titanium dioxide content is 5 to 20 wt. percent, based on the weight of the support.

## Patentansprüche

1. Verfahren zur Herstellung eines Acetylpyridins der Formel 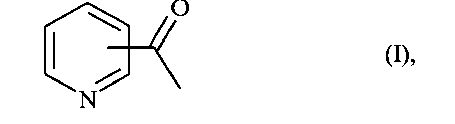 durch Umsetzung eines Pyridincarbonsäureesters der Formel 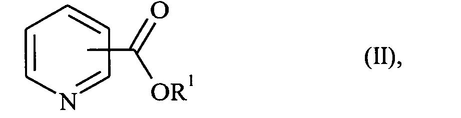 worin R¹ C₁₋₆-Alkyl ist, mit Essigsäure in der Gasphase in Gegenwart eines Katalysators, dessen aktives Material Titandioxid und wenigstens ein Alkali- oder Erdalkalimetalloxid umfasst, wobei der Katalysator einen Aluminiumoxid/Siliciumoxid-Träger mit einer scheinbaren Porosität von mindestens 50% umfasst.

2. Verfahren nach Anspruch 1, worin das hergestellte Acetylpyridin (I) 3-Acetylpyridin und der Pyridincarbonsäureester (II) ein (C₁₋₆-Alkyl)nicotinat ist.

3. Verfahren nach Anspruch 1 oder 2, worin der Pyridincarbonsäureester (II) bei Atmosphärendruck einen Siedepunkt besitzt, der um mehr als 20 K höher als der Siedepunkt des Acetylpyridins (I) ist.

4. Verfahren nach einem der Ansprüche 1 bis 3, worin die Reaktionstemperatur zwischen 350 und 450 °C liegt.

5. Verfahren nach einem der Ansprüche 1 bis 4, worin das Gewichtsverhältnis von Aluminiumoxid zu Siliciumoxid im Katalysatorträger zwischen 70:30 und 90:10 und vorzugsweise zwischen 75:25 und 85:15 liegt.

6. Verfahren nach einem der Ansprüche 1 bis 5, worin die scheinbare Porosität des Katalysatorträgers zwischen 60 und 70% liegt.

7. Verfahren nach einem der Ansprüche 1 bis 6, worin die Packungsdichte des Katalysatorträgers zwischen 600 und 1000 kg/m³ und vorzugsweise zwischen 600 und 800 kg/m³ liegt.

8. Verfahren nach einem der Ansprüche 1 bis 7, worin der Titandioxidgehalt des Katalysators, bezogen auf das Gewicht des Trägers, 5 bis 20 Gew.-% beträgt.

9. Verfahren nach einem der Ansprüche 1 bis 4, worin der Katalysator erhalten wurde durch
(i) Imprägnieren eines Aluminiumoxid/Siliciumoxid-Trägers mit einer scheinbaren Porosität von mindestens 50% mit einer Lösung von Titantetrachlorid in wässriger Salzsäure zur Gewinnung eines ersten Katalysatorvorläufers,
(ii) Trocknen,
(iii) Kalzinieren,
(iv) Imprägnieren des kalzinierten ersten Katalysatorvorläufers mit einer Lösung oder Suspension eines Hydroxids und/oder Oxids eines Alkali- und/oder Erdalkalimetalls zur Gewinnung eines zweiten Katalysatorvorläufers,
(v) Trocknen und
(vi) Kalzinieren des getrockneten zweiten Katalysatorvorläufers.

10. Verfahren nach Anspruch 9, worin das Gewichtsverhältnis von Aluminiumoxid zu Siliciumoxid im Träger zwischen 70:30 und 90:10 und vorzugsweise zwischen 75:25 und 85:15 liegt.

11. Verfahren nach Anspruch 9 oder 10, worin die scheinbare Porosität des Katalysatorträgers zwischen 60 und 70% liegt.

12. Verfahren nach einem der Ansprüche 9 bis 11, worin der Titandioxidgehalt, bezogen auf das Gewicht des Trägers, 5 bis 20 Gew.-% beträgt.

## Revendications

1. Procédé de préparation d'une acétylpyridine de formule 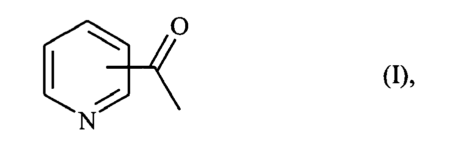 en faisant réagir un ester pyridinecarboxylique de formule 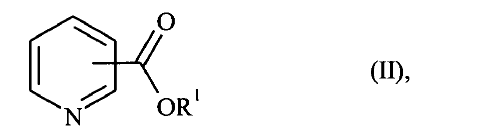 dans laquelle R¹ représente un groupe alkyle de C₁ à C₆, avec de l'acide acétique dans la phase gazeuse en présence d'un catalyseur dont le matériau actif est composé de dioxyde de titane et d'au moins un oxyde de métal alcalin ou de métal alcalino-terreux, le catalyseur comprenant un support d'alumine-silice dont la porosité apparente est d'au moins 50%.

2. Procédé selon la revendication 1, dans lequel l'acétylpyridine (I) préparée est de la 3-acétylpyridine et l'ester pyridinecarboxylique (II) est du nicotinate d'alkyle de C₁ à C₆.

3. Procédé selon l'une des revendications 1 et 2, dans lequel l'ester pyridinecarboxylique (II) a un point d'ébullition à la pression atmosphérique qui est plus de 20 K supérieur au point d'ébullition de l'acétylpyridine (1).

4. Procédé selon l'une quelconque des revendications 1 à 3, dans lequel la température de la réaction se situe entre 350 et 450 °C.

5. Procédé selon l'une quelconque des revendications 1 à 4, dans lequel le rapport en poids de l'alumine à la silice dans le support du catalyseur se situe entre 70:30 et 90:10, de préférence entre 75:25 et 85:15.

6. Procédé selon l'une quelconque des revendications 1 à 5, dans lequel la porosité apparente du support du catalyseur se situe entre 60 et 70 %.

7. Procédé selon l'une quelconque des revendications 1 à 6, dans lequel la densité de tassement du support du catalyseur se situe entre 600 et 1 000 kg/m³, de préférence entre 600 et 800 kg/m³.

8. Procédé selon l'une quelconque des revendications 1 à 7, dans lequel la teneur en dioxyde de titane du catalyseur varie de 5 à 20 % en poids, basée sur le poids du support.

9. Procédé selon l'une quelconque des revendications 1 à 4, dans lequel le catalyseur est obtenu
(i) en imprégnant le support d'alumine-silice d'une porosité apparente d'au moins 50 % avec une solution de tétrachlorure de titane dans de l'acide chlorhydrique aqueux pour obtenir un premier précurseur de catalyseur,
(ii) en séchant,
(iii) en calcinant,
(iv) en imprégnant le premier précurseur de catalyseur calciné avec une solution ou une suspension d'un hydroxyde et/ou d'un oxyde d'un métal alcalin et/ou d'un métal alcalino-terreux pour obtenir un second précurseur de catalyseur,
(v) en séchant et
(vi) en calcinant le second précurseur de catalyseur séché.

10. Procédé selon la revendication 9, dans lequel le rapport en poids de l'alumine à la silice dans le support se situe entre 70:30 et 90:10, de préférence entre 75:25 et 85:15.

11. Procédé selon l'une des revendications 9 et 10, dans lequel la porosité apparente du support du catalyseur se situe entre 60 et 70 %.

12. Procédé selon l'une quelconque des revendications 9 à 11, dans lequel la teneur en dioxyde de titane varie de 5 à 20 % en poids, basée sur le poids du support.
